# EUROPEAN PATENT SPECIFICATION

(11) **EP 1 971 585 B1**
(45) Date of publication and mention of the grant of the patent: **09.03.2011**
(21) Application number: 06842191.6
(22) Date of filing: 08.12.2006
(51) Int. Cl.: C07D 295/088

(54) **PROCESS FOR THE PREPARATION OF A PHARMACEUTICAL INTERMEDIATE**
VERFAHREN ZUR HERSTELLUNG EINES PHARMAZEUTISCHEN ZWISCHENPRODUKTS
PROCEDE DE PREPARATION D'UN INTERMEDIAIRE PHARMACEUTIQUE

(30) Priority: 08.12.2005 HU 0501138
(43) Date of publication of application: 24.09.2008
(73) Proprietor: Egis Gyógyszergyár Nyilvánosan Múködö Részvénytársaság, 1106 Budapest (HU)
(72) Inventor: TRINKA, Péter, H-1116 Budapest (HU); REITER, József, H-1022 Budapest (HU); BARTHA, Ferenc, H- Tiszavasvári (HU); KATONA, Zoltán, H-3300 Eger (HU); VERECZKEYNÉ, Donáth, Györgyi, H-1034 Budapest (HU); NAGY, Kálmán, H-1025 Budapest (HU); PONGÓ, László, H-2144 Kerepes (HU); MEZEI, Tibor, H-1221 Budapest (HU)
(74) Representative: Beszédes, Stephan G.
(86) International application number: PCT/HU2006/000108
(87) International publication number: WO 2007/066162

(56) References cited:
- EP-A2- 0 952 153
- WO-A-01/40211
- GB-A- 2 225 321
- OPALKA C J ET AL: "A NOVEL SYNTHESIS OF THE ENANTIOMERS OF AN ANTIHISTAMINE DRUG BY PIPERAZINE FORMATION FROM A PRIMARY AMINE" SYNTHESIS, GEORG THIEME VERLAG, STUTTGART, DE, vol. 7, no. 7, July 1995 (1995-07), pages 766-768, XP000979124 ISSN: 0039-7881

## Description

The present invention relates to the preparation of {2-[4-(α-phenyl*-p*-chlorobenzyl)-piperazin-1-yl]-ethoxy}-acetic acid-*N,N-*dimethylamide of the Formula (I), 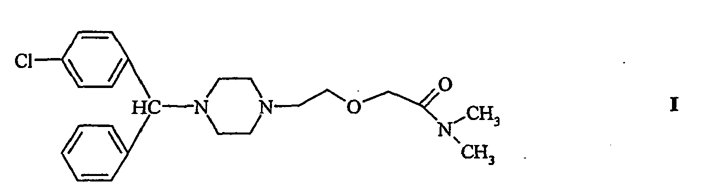 optical isomers and salts thereof. The compound of the Formula (I) is an important intermediate of the pharmaceutical active ingredient {2-[4-(a*-*phenyl*-p*-chlorobenzyl)-piperazine-1-yl]-ethoxy}-acetic acid, which is known by the International Nonpropriatory Name cetirizine. Cetirizine is a non-sedating antihistamine type pharmaceutical ingredient.

The compound (-)-{2-[4-(a-phenyl*-p*-chlorobenzyl)-piperazin-1-yl]-ethoxy}-acetic acid-*N,N*-dimethylamide of the Formula (I) is the starting material in the preparation process of the (-)-{2-[4-(α-phenyl-*p*-chlorobenzyl)-piperazin-1-yl]-ethoxy}-acetic acid of the Formula (VI), 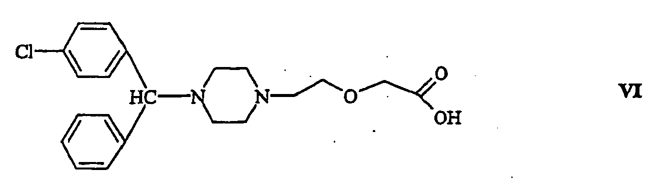 which is also a pharmaceutically active ingredient known by the International Nonproprietary Name levocetirizine. Levocetirizine belongs to the group of non-sedating antihistamine type pharmaceutical active ingredients. Cetirizine and levocetirizine have become known from United States Patents Nos. 5 627 183. and 5 698 558.

A process for the preparation of cetirizine has been disclosed for the first time in European Patent No. 58146. Cetirizine was obtained by the hydrolysis of {2-[4-(α-phenyl*-p*-chlorobenzyl)-piperazin-1-yl]-ethoxy}-acetamide of the Formula (VII).

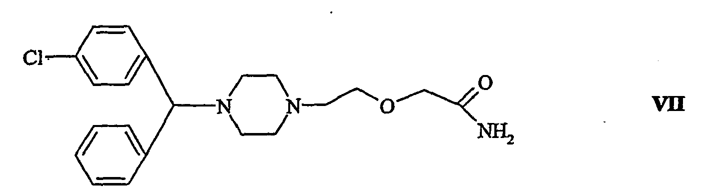

Said {2-[4-(α-phenyl*-p-*chlorobenzyl)-piperazin-1-yl]-ethoxy}-acetamide of the Formula (VII) was prepared by reacting [1-(α-phenyl*-p-*chlorobenzyl)-piperazine of the Formula (II) with 2-chloroethoxy-acetamide of the Formula (VIII) 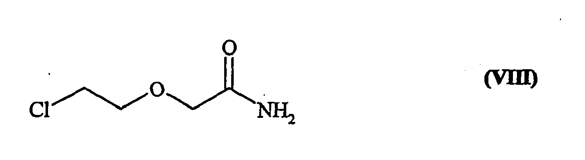 in xylene solvent in presence of anhydrous sodium carbonate.

The disadvantage of the above mentioned process resides in the fact that during the reaction the alkylation of the amino group of 2-chloroethoxy-acetamide also takes place as side reaction. The side products formed in the above reaction can not be recoved from the product by a simple process. Removal of said side products and purification of the product requires costly purification process which also results in the significant loss of yield.

International Publication Document WO 01/40211 discloses a process for the preparation of {2-[4-(a-phenyl*-p-*chlorobenzyl)-piperazine-1-yl]-ethoxy}-acetic acid-*N,N*-dimethylamide of the Formula (I) from the compound 2-[4-(α-phenyl*-p-*chlorobenzyl)-piperazine-1-yl]-ethanol of the Formula (IV) 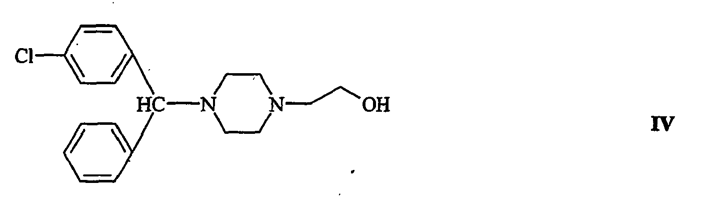 by converting said substituted ethanol derivative into its alkaline metal salt in an indifferent solvent and reacting the thus obtained salt with chloroacetic acid-*N,N*-dimethylamide of the Formula (V).

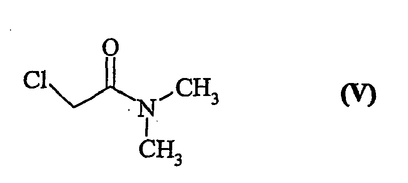

The product {2-[4-(α-phenyl*-p-*chlorobenzyl)-piperazin-1-yl]-ethoxy}-acetic acid-*N,N*-dimethylamide of the Formula (1) is obtained in the form of dihydrochloride salt, which is directly used as starting material in the process for the preparation of cetirizine.

The disadvantage of the above mentioned process resides in the fact that the *O*-alkylation of the compound 2-[4-(α-phenyl*-p-*chlorobenzyl)-piperazin-1-yl]-ethanol of the Formula (IV) can be carried out in a solution which is free from water and alcohols, which requires using special equipment as well as moisture-free reagents.

The objective of our research work was to develop a process for the preparation of {2-[4-(a-phenyl*-p-*chlorobenzyl)-piperazin-1-yl]-ethoxy}-acetic acid-*N,N*-dimethyl-amide of the Formula (I) suitable for the production of the end product cetirizine in pharmacopoeial quality without the use of expensive reagents, complicated and costly equipment and without complicated operations (e.g. water and moisture removal, purification of the product from side products).

The above objective is solved by the process according to the present invention.

The basis of the present invention is the surprising recognition that the above mentioned drawbacks can be eliminated if the compound {2-[4-(a-phenyl*-p-*chlorobenzyl)-piperazin-1-yl]-ethoxy}-acetic acid-*N,N*-dimethyl-amide of the Formula (I) is prepared by reacting 1-(α-phenyl*-p-*chlorobenzyl)-piperazine of the Formula (II) 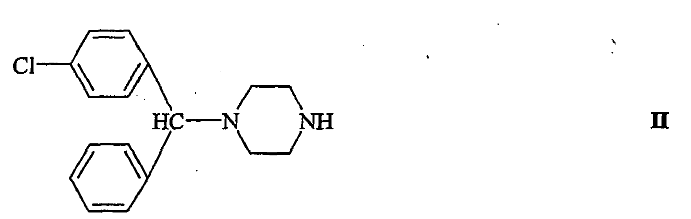 and (2-chloroethoxy)-acetic acid-*N,N*-dimethylamide of the Formula (III) 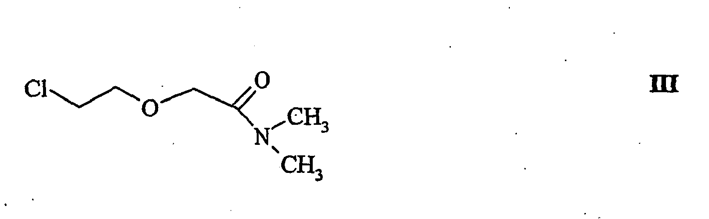 in an indifferent solvent and in the presence of an acid-binding agent and a catalyst. {2-[4-(α-phenyl*-p-*chlorobenzyl)-piperazin-1-yl]-ethoxy}-acetic acid-*N,N*-dimethylamide of the Formula (I) obtained in said process is suitable for its direct coversion into cetirizine. A further advantage of the process resides in the fact that by using the single enantiomer of the starting compound (-)-1-(α-phenyl*-p-*chlorobenzyl)-piperazine of the Formula (II), levocetirizine can be prepared.

In the above described process according to the present invention, only small amount of side products are formed and no moisture-free operation conditions are required.

A particularly surprising feature of the process resides in the fact that during the alkaline hydrolysis of (-)-{2-[4-(α-phenyl*-p-*chlorobenzyl)-piperazin-1-yl]-ethoxy}-acetic acid-*N,N-*dimethylamide no racemization takes place, therefore said alkaline hydrolysis results in optically pure (-)-{2-[4-(α-phenyl*-p-*chlorobenzyl)-piperain-1-yl]-ethoxy}-acetic acid [levocetirizine] free from the (+) isomer.

According to the present invention, alkylation of 1-(α-phenyl*-p-*chlorobenzyl)-piperazine of the Formula (II) with (2-chloroethoxy)-acetic acid-*N,N*-dimethylamide of the Formula (III) is carried out in a protic or aprotic, polar or apolar solvent, such as aliphatic alcohols comprising 1 to 4 carbon atoms, acetonitrile, toluene, dioxane, acetone, preferably in toluene.

For the neutralization of the acid formed in the reaction, a weak organic or inorganic base, such as an alkali metal or alkali earth metal carbonate or hydrogencarbonate, preferably sodium carbonate, pyridine or a tertiary aliphatic amine wherein the alkyl group comprises 2 to 6 carbon atoms, preferably, triethylamine can be used.

As catalyst, an alkali metal halogenide, such as alkali metal iodide or alkali metal bromide, e.g. lithium, sodium or potassium iodide, preferably potassium iodide can be used.

The reaction is carried out between 50°C and the boiling temperature of the solvent, preferably, between 80°C and the boiling temperature of the solvent, the most advantageously at the boiling temperature of the solvent.

The product can be isolated by methods known *per se* from the prior art. Especially advantageous method is obtaining the product in the form of its dihydrochloride.

The starting substance of the process according to the present invention, 1-(α-phenyl*-p-*chlorobenzyl)-piperazine of the Formula (II) and optical isomers thereof are known compounds [Yakugaku Zasshi, 74, 1954, 1049; Chem. Abstr., 1955, 11666; GB Patent No. 2 225 321].

The second starting substance of the process according to the present invention, 2-chloroethoxy-acetic acid-*N*,*N*-dimethylamide is known from German Patent No. 2 150 075.

Further details of the present invention are provided in the following examples without limiting the scope of protection to said examples.

### Example 1

### (±)-{2-[4-(a-phenyl-p-chlorobenzyl)-piperazin-1-yl]-ethoxy}-acetic acid-N,N-dimethylamide

14.4 g (0.05 mole) of (±) 1-(α-phenyl*-p-*chlorobenzyl)-piperazine are dissolved in 120 ml toluene and 15 g of anhydrous sodium carbonate and 0.7 g of potassium iodide are added. The reaction mixture is heated to approximately 110°C with stirring and at this temperature, 8.8 g (0.053 mole) of raw 2-chloroethoxy-acetic acid-*N,N*-dimethylamide is added dropwise in one hour. After the addition, the reaction mixture is heated and stirred at the above temperature for 24 hours.

The reaction mixture is cooled, 20 g of crushed ice are added to it and the pH of the mixture is adjusted to 6.4 by addition of approximately 1.5 ml of concentrated hydrochloric acid. The layers are separated, to the toluene layer 25 g of crushed ice are added and its pH is adjusted to 3.8 by addition of approximately 3 ml of concentrated hydrochloric acid. The layers are separated, 50 ml of dichloromethane are added to the aqueous layer and its pH is adjusted to 7-8 by addition of approximately 4.5 ml 40 % by weight sodium hydroxide solution. The dichloromethane layer is separated, dried over anhydrous sodium sulphate, filtered and the solvent evaporated *in vacuo.*

By this process, 18.4 g (88.2 %) of a viscous, oily product are obtained (melting temperature 183-190°C). The purity of the product is greater than 98 % by weight as determined by high performance liquid chromatography. The product is suitable as starting material for the production of pharmacopoeial quality cetirizine complying with the specifications set forth in European Pharmacopoeia 1997:1084.

According to the above mentioned specifications, the amount of the total impurities must not exceed 0.5 % by weight and the amount of any single impurity shall be less than 0.1 % by weight.

If desired, the free base can be converted into its dihydrochloride salt using 2-propanol as solvent by reacting the free base with 25 % by weight isopropanolic hydrochloric acid solution.

### Example 2

### [Reference Example]

### (±){2-[4-(α-phenyl-p-chlorobenzyl)-piperazin-1-yl]-ethoxy}-acetic acid dihydrochloride

48.9 g (0.1 mole) of (+){2-[4-(α-phenyl*-p-*chlorobenzyl)-piperazin-1-yl]-ethoxy}-acetic acid-*N*,*N*-dimethylamide dihydrochloride prepared according to the process of Example 1 are dissolved in 170 ml water and the resulting solution is mixed with 100 ml of 40 % by weight aqueous sodium hydroxide solution. The resulting suspension is boiled for three hours during continous bubbling with nitrogen. The reaction mixture obtained is allowed to cool to 40 °C, diluted with 250 ml of water and acidified with approximately 60 ml of concentrated hydrochloric acid to pH 3.8. The acidic solution is extracted with 200 ml and 100 ml dichloromethane, respectively. The dichloromethane layers are combined, the solvent is evaporated and the residue is dissolved in 25 ml of water. The aqueous solution is mixed with 12 ml of concentrated hydrochloric acid and evaporated to dryness in vacuo. The thick oily residue is dissolved in 25 ml of acetone, the solution is mixed with a further portion of 300 ml acetone and stirred for one hour. The precipitated crystalline product is filtered off, washed with acetone and diethylether and dried in vacuo. Thus 39.4 g (85.5 %) pure title compound are obtained, melting temperature 225.5-228 °C. The product thus obtained complies with all requirements of the European Pharmacopoeia 3, 1997:1084.

### Example 3

### [Reference Example]

### (±){2-[4-(α-phenyl-p-chlorobenzyl)-piperazin-1-yl]-ethoxy}-acetic acid dihydrochloride

The process of the Example 2 is carried out with the difference that instead of 48.9 g (0.1 mole) (±){2-[4-(α-phenyl*-p-*chlorobenzyl)-piperazin-1-yl]-ethoxy}-acetic acid-*N,N*-dimethylamide dihydrochloride, 41.7 g (0.1 mole) (±){2-[4-(α-phenyl*-p-*chlorobenzyl)-piperazin-1-yl]-ethoxy}-acetic acid-*N,N-*dimethylamide are used. Yield 38.1 g (82.1 %) of pure title compound (melting temperature 226-228 °C), which complies with all quality requirements set forth in European Pharmacopoeia 3, 1997:1084.

### Example 4

### (-){2-[4-(a-phenyl-p-chlorobenzyl)-piperazin-1-yl]-ethoxy}-acetic acid-N,N-dimethylamide

The process of Example 1 is followed with the difference that instead of (±)-1-(α-phenyl*-p-*chlorobenzyl)-piperazine, (-)-1-(α-phenyl*-p-*chlorobenzyl)-piperazine having enantiomeric purity greater than 99% is used. Yield, 18.2 g (87.3 %) of a viscous oily product having the purity higher than 98% determined by high performance liquid chromatography. Optical purity of the product determined by chiral high performance liquid chromatography is higher than 99%. Optical rotation, [α]_{D}²⁰ =-6.4° (c= 0.8 g/ 10 cm³. ethanol).

The purity of the product is determined by high performance liquid chromatography (HPLC) using a Chiralpak AD column and the mobile phase comprising 2-propanol-hexane 25:75 (v/v).

### Example 5

### [Reference Example]

### (+){2-[4-(a-phenyl-p-chlorobenzyl)-piperazine-1-yl]-ethoxy}-acetic acid dihydrochloride

Process of Example 2 is followed with the difference that instead of (±){2-[4-(α-phenyl*-p-*chlorobenzyl)-piperazin-1-yl]-ethoxy}-acetic acid-*N,N*-dimethylamide dihydrochloride, (-){2-[4-(α-phenyl*-p-*chlorobenzyl)-piperazine-1-yl]-ethoxy}-acetic acid-*N,N-*dimethylamide having optical purity greater than 99% is used and the reaction is carried out on 0.02 mole scale. Yield, 7.3 g (79.4 %) of the title compound having optical rotation [a]₃₆₅²⁰ = +12.2°. The purity of the product as determined by high performance liquid chromatography is higher than 98%. The optical purity determined by chiral high performance liquid chromatography of the product is greater than 99 %.

The optical purity of the product is determined by HPLC using a Chiracell OD-R column and the mobile phase comprising 2-propanol-hexane 32:68 (v/v) acetonitrile-0.5 M sodium perchlorate.

### Example 6

### (+){2-[4-(α-phenyl-p-chlorobenzyl)-piperazin-1-yl]-ethoxy}-acetic acid-N,N-dimethylamide

The process according to Example 1 is followed with the difference that instead of using (±)-1-(a-phenyl*-p-*chlorobenzyl)-piperazine, (+)-1-(α-phenyl*-p-*chlorobenzyl)-piperazine of enantiomeric purity higher than 99% is used. Yield: 17.5 g (83.8 %) of a viscous oily product, the purity thereof is higher than 98% as determined by high performance liquid chromatography. The optical purity of the product as determined by chiral high performance liquid chromatography is higher than 99%. Optical rotation [α]_{D}²⁰= + 6.3° (c = 0.8 g/10 cm³ ethanol).

### Example 7

### [Reference Example]

### (-)-{2-[4-(a-phenyl-p-chlorobenzyl)-piperazin-1-yl]-ethoxy}-acetic acid dihydrochloride

The title compound is prepared according to the process of Example 2 with the difference that instead of (±)-{2-[4-(α-phenyl-*p*-chlorobenzyl)-piperazin-1-yl]-ethoxy}-acetic acid-*N,N-*dimethylamide dihydrochloride, (+)-{2-[4-(α-phenyl*-p-*chlorobenzyl)-piperazin-1-yl]-ethoxy}-acetic acid-*N,N-*dimethylamide having optical purity higher than 99% is used as starting material and the reaction is carried out on a 0.02 molar scale. Yield: 6.9 g (75.0 %), optical rotation [α]₃₆₅²⁰ = - 12.4°, purity by high performance liquid chromatography is higher than 98 %. The optical purity of the product determined by chiral high performance liquid chromatography is higher than 99 %.

### Example 8

### (±){2-[4-(α-phenyl-p-chlorobenzyl)-piperazin-1-yl]-ethoxy}-acetic acid-N,N-dimethylamide

The title compound is prepared according to the process of Example 1 with the difference that instead of 120 ml toluene solvent, 100 ml acetonitrile are used and the reaction is carried out at the boiling temperature of acetonitrile for a period of 24 hours. Yield 10.2 g (48.9 %) of a viscous, honey-like product, which has purity higher than 98% as determined by high performance liquid chromatography and which is suitable for direct conversion into cetirizin which complies with the specification set forth in European Pharmacopoeia.

### Example 9

### (±)-{2-[4-(a-phenyl-p-chlorobenzyl)-piperazin-1-yl]-ethoxy}-acetic acid-N,N-dimethylamide

The title compound is prepared according to the process of Example 1 with the difference that instead of 120 ml toluene solvent, 150 ml of acetone are used and the reaction is carried out by boiling the reaction mixture for 48 hours at the boiling temperature of acetone. Yield 9.4 g (45.1 %) of a honey-like product, which has purity higher than 95% on the basis of high performance liquid chromatographic analysis and which is suitable for direct conversion into cetirizin which complies with the specification of European Pharmacopoeia.

### Example 10

### (±)-{2-[4-(α-phenyl-p-chlorobenzyl)-piperazin-1-yl]-ethoxy}-acetic acid-N,N-dimethylamide

The title compound is prepared according to the process of Example 1 with the difference that instead of 120 ml of toluene solvent, 120 ml of dioxane are used and the reaction is completed by boiling the reaction mixture for 24 hours at the boiling temperature of dioxane. Yield 10.8 g of a (51.8 %) viscous, honey-like product, which has purity determined by high performance liquid chromatography higher than 98% and which is suitable for direct transformation into cetirizine of pharmacopoeial quality.

### Example 11

### (±)-{2-[4-(α-phenyl-p-chlorobenzyl)-piperazine-1-yl]-ethoxy}-acetic acid-N,N-dimethylamide

The title compound is prepared according to the process of Example 1 with the difference that instead of 120 ml toluene solvent, 100 ml of acetonitrile and in place of anhydrous sodium carbonate acid binding agent, 10 ml of triethylamine are used and that the reaction is made complete by heating the reaction mixture at 80 °C for 72 hours. Yield 8.2 g (39.3 %) of a honey-like product having purity higher than 98% as determined by high performance liquid chromatography which is directly suitable for transformation into cetirizine complying with the requirements of the European Pharmacopoeia.

## Claims

1. Process for the preparation of {2-[4-(α-phenyl*-p-*chlorobenzyl)-piperazin-1-yl]-ethoxy}-acetic acid-*N,N-*dimethylamide of the Formula (I) 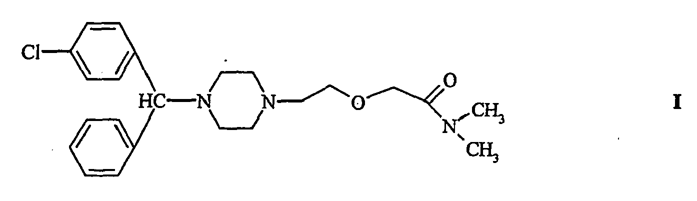 and enantiomers thereof, which comprises reacting 1-(α-phenyl*-p-*chlorobenzyl)-piperazine of the Formula (II) 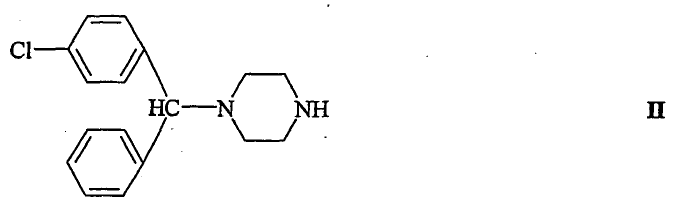 or an enantiomer thereof with β-chloroethoxy-acetic acid-*N,N*-dimethylamide of the Formula (III) 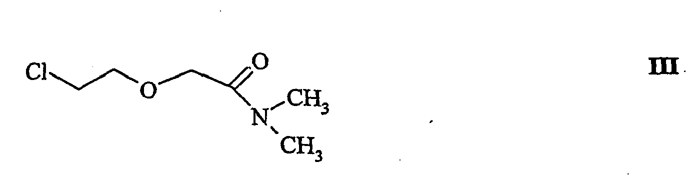 in an indifferent solvent in presence of a catalyst and an acid-binding agent.

2. The process according to claim 1, **characterized in that** an aliphatic alcohol comprising 1 to 4 carbon atoms, acetonitrile, toluene, dioxane or aceton or a mixture thereof is used as solvent.

3. The process according to claim 1, **characterized in that** an inorganic or organic base is used as acid-binding agent.

4. The process according to claim 3, **characterized in that** an inorganic base selected from alkali metal carbonates or alkali earth metal carbonates is used as acid-binding agent.

5. The process according to claim 3, **characterized in that** an organic base selected from triethylamine or pyridine is used as acid-binding agent.

6. The process according to claim 1, **characterized in that** an alkali metal iodide or alkali metal bromide is used as catalyst.

7. The process according to claim 6, **characterized in that** as alkali metal iodide catalyst potassium iodide is used.

8. The process according to claim 1, **characterized in that** the reaction is carried out by heating the reaction mixture at a temperature between 50°C and the boiling temperature of the solvent.

9. The process according to claim 8, **characterized in that** the heating of the reaction mixture is carried out at a temperature between 80° C and the boiling temperature of the solvent.

10. The process according to claim 9, **characterized in that** the heating of the reaction mixture is carried out at the boiling temperature of the solvent.

11. The process according to claim 1, **characterized in that** (-)-{1-(α-phenyl*-p-*chlorobenzyl)-piperazine of the Formula (II) is used as starting material.

## Patentansprüche

1. Verfahren zur Herstellung von {2-[4-(α-Phenyl*-p-*chlorbenzyl)-piperazin-1-yl]-ethoxy}-essigsäure-*N,N*-dimethylamid der Formel (I) 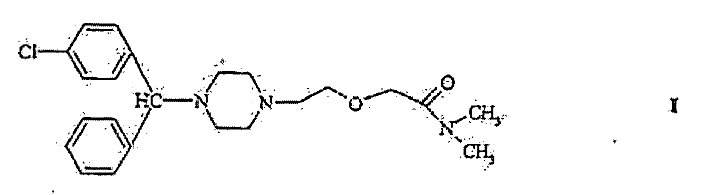 und Enantiomeren davon, welches das Umsetzen von 1-(α-Phenyl*-p-*chlorbenzyl)-piperazin der Formel (II) 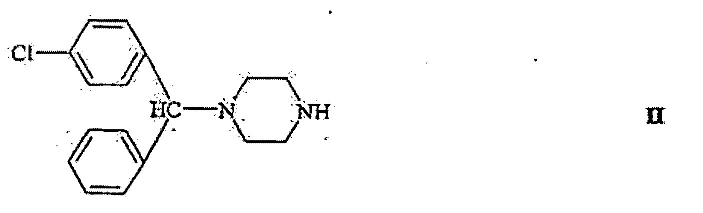 oder eines Enantiomeren davon mit β-Chlorethoxy-essigsäure-*N,N*-dimethylamid der Formel (III) 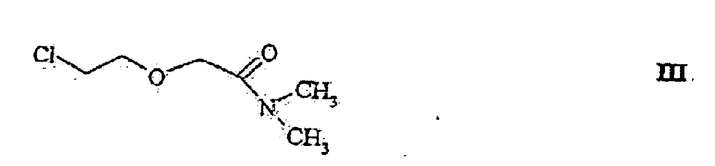 in einem indifferenten Lösungsmittel in Gegenwart eines Katalysators und eines säurebindendes Mittel umfasst.

2. Verfahren gemäß Anspruch 1, **dadurch gekennzeichnet, dass** ein aliphatischer Alkohol, welcher 1 bis 4 Kohlenstoffatome umfasst, Acetonitril, Toluol, Dioxan oder Aceton oder eine Mischung davon als Lösungsmittel verwendet wird.

3. Verfahren gemäß Anspruch 1, **dadurch gekennzeichnet, dass** eine anorganische oder organische Base als säurebindendes Mittel verwendet wird.

4. Verfahren gemäß Anspruch 3, **dadurch gekennzeichnet, dass** eine anorganische Base, die aus Alkalimetallcarbonaten oder Erdalkalimetallcarbonaten gewählt wird, als säurebindendes Mittel verwendet wird.

5. Verfahren gemäß Anspruch 3, **dadurch gekennzeichnet, dass** eine organische Base, die aus Triethylamin oder Pyridin gewählt wird, als säurebindendes Mittel verwendet wird.

6. Verfahren gemäß Anspruch 1, **dadurch gekennzeichnet, dass** ein Alkalimetalliodid oder Alkalimetallbromid als Katalysator verwendet wird.

7. Verfahren gemäß Anspruch 6, **dadurch gekennzeichnet, dass** als Alkalimetalliodid-Katalysator Kaliumiodid verwendet wird.

8. Verfahren gemäß Anspruch 1, **dadurch gekennzeichnet, dass** die Reaktion durch Erhitzen der Reaktionsmischung bei einer Temperatur zwischen 50°C und der Siedetemperatur des Lösungsmittels durchgeführt wird.

9. Verfahren gemäß Anspruch 8, **dadurch gekennzeichnet, dass** das Erhitzen der Reaktionsmischung bei einer Temperatur zwischen 80°C und der Siedetemperatur des Lösungsmittels durchgeführt wird.

10. Verfahren gemäß Anspruch 9, **dadurch gekennzeichnet, dass** das Erhitzen der Reaktionsmischung bei der Siedetemperatur des Lösungsmittels durchgeführt wird.

11. Verfahren gemäß Anspruch 1, **dadurch gekennzeichnet, dass** (-)-{1-(α-Phenylp-chlorbenzyl)-piperazin der Formel (II) als Ausgangsmaterial verwendet wird.

## Revendications

1. Procédé pour la préparation de *N,N*-diméthylamide de l'acide {2-[4-(α-phényl*-p-*chlorobenzyl)-pipérazin-1-yl]-éthoxy}-acétique de formule (I) 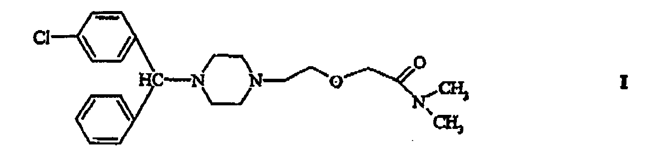 et d'énantiomères de celui-ci, qui comprend la réaction de 1-(α-phényl*-p-*chloro-benzyl)-pipérazine de formule (II) 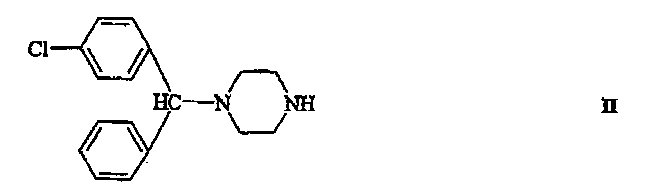 ou d'un énantiomère de celle-ci, avec du *N,N*-diméthylamide de l'acide β-chloro-éthoxy-acétique de formule (III) 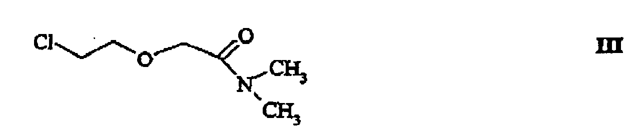 dans un solvant indifférent en présence d'un catalyseur et d'un agent liant un acide.

2. Procédé selon la revendication 1, **caractérisé en ce qu'**un alcool aliphatique comprenant 1 à 4 atomes de carbone, de l'acétonitrile, du toluène, du dioxane ou de l'acétone ou un mélange de ceux-ci est utilisé en tant que solvant.

3. Procédé selon la revendication 1, **caractérisé en ce qu'**une base inorganique ou organique est utilisée en tant qu'agent liant un acide.

4. Procédé selon la revendication 3, **caractérisé en ce qu'**une base inorganique choisie parmi les carbonates de métaux alcalins ou les carbonates de métaux alcalino-terreux est utilisée en tant qu'agent liant un acide.

5. Procédé selon la revendication 3, **caractérisé en ce qu'**une base organique choisie parmi la triéthylamine ou la pyridine est utilisée en tant qu'agent liant un acide.

6. Procédé selon la revendication 1, **caractérisé en ce qu'**un iodure de métal alcalin ou un bromure de métal alcalin est utilisé en tant que catalyseur.

7. Procédé selon la revendication 6, **caractérisé en ce qu'**un catalyseur iodure de métal alcalin, l'iodure de potassium, est utilisé.

8. Procédé selon la revendication 1, **caractérisé en ce que** la réaction est effectuée en chauffant le mélange réactionnel à une température entre 50 °C et la température d'ébullition du solvant.

9. Procédé selon la revendication 8, **caractérisé en ce que** le chauffage du mélange réactionnel est effectué à une température entre 80 °C et la température d'ébullition du solvant.

10. Procédé selon la revendication 9, **caractérisé en ce que** le chauffage du mélange réactionnel est effectué à la température d'ébullition du solvant.

11. Procédé selon la revendication 1, **caractérisé en ce qu'**une (-)-{1-(α-phényl*-p-*chloro-benzyl)-pipérazine de formule (II) est utilisée en tant que matière de départ.
